Europäisches Patentamt

European Patent Office

Office européen des brevets

(11) Numéro de publication: **0 091 349**
**B1**

# (12) FASCICULE DE BREVET EUROPÉEN

(45) Date de publication du fascicule du brevet:
17.12.86

(51) Int. Cl.⁴: **A 61 G 7/04**

(21) Numéro de dépôt: **83400624.9**

(22) Date de dépôt: **25.03.83**

(54) **Lit thérapeutique à lit fluidisé.**

(30) Priorité: **25.03.82 FR 8205064**

(43) Date de publication de la demande:
**12.10.83 Bulletin 83/41**

(45) Mention de la délivrance du brevet:
**17.12.86 Bulletin 86/51**

(84) Etats contractants désignés:
**AT BE CH DE GB IT LI SE**

(56) Documents cité:
**EP-A-0 038 695**
**CH-A-486 199**
**DE-A-2 358 174**
**DE-A-2 656 465**
**FR-A-1 516 528**
**FR-A-2 168 745**
**GB-A-1 564 271**
**US-A-2 904 462**

(73) Titulaire: **Lacoste, François René, 35 Boulevard
d'Inckermann, F-92200 Neuilly (FR)**

(72) Inventeur: **Lacoste, François René, 35 Boulevard
d'Inckermann, F-92200 Neuilly (FR)**

(74) Mandataire: **Viard, Jean, Cabinet VIARD 28 bis,
avenue Mozart, F-75016 Paris (FR)**

EP 0 091 349 B1

LIBER, STOCKHOLM 1986

## Description

La présente invention a pour objet un lit fluidisé à usage thérapeutique destiné en particulier, mais non exclusivement, à recevoir, des malades souffrant de brûlures ou d'escarres.

Les lits de ce type sont connus et leur usage se généralise rapidement dans les hôpitaux. Ils sont décrits dans le brevet FR.A. 1.516.528 du 17 Mars 1967. Chacun d'eux comprend une cuve remplie d'une matière granulaire et un dispositif destiné à insuffler un gaz sous pression de sorte que la matière granulaire soit fluidifiée sans toutefois être chassée hors de la cuve. La partie inférieure de la cuve est constituée par un écran diffuseur suffisamment poreux pour permettre le passage du gaz tout en empêchant le passage de la matière granulaire.

Pratiquement, la matière granulaire est constituée par des microbilles de verre dont le diamètre est de l'ordre de 75 microns et la densité du milieu obtenue après fluidisation est de l'ordre de 1,6 à 1,7. Le patient est ainsi supporté sur le lit comme si il flottait sur un liquide dense. Il en résulte une pression uniformément répartie du corps de celui-ci sur le milieu granulaire et par suite un sentiment de confort pour le malade. Afin d'éviter le contact des microbilles avec la peau du malade, un drap poreux permettant le passage du gaz (pratiquement de l'air) tout en évitant le passage des billes, est simplement posé sur la surface supérieure de la couche granulaire.

Compte tenu des risques d'infection des plaies ouvertes de malades traités sur ces lits, des études sytématiques ont été conduites sur l'environnement bactériologique créé par ces lits. Ces études ont montré que ces lits étaient bactériostatiques et même souvent bactéricides, pour la plupart des souches microbiennes rencontrées. L'explication de cette caractéristique favorable semble être, d'une part l'effet dissécant du courant d'air et, d'autre part, la tendance modérément basique provenant des ions(+)sodium à la surface du verre dont les billes sont actuellement constituées. Un certain rôle semble également être joué par la poussière de verre provenant de l'usure des billes. Ces caractéristiques favorables sont cependant contrebalancées, d'une part, par la présence d'une fine couche de matériau antifriction recouvrant les billes pour faciliter leur fluidisation ce qui supprime l'essentiel du contract entre le verre et le milieu extérieur, et, d'autre part, par le fait que la poussière de verre se trouve en proportions variables selon les fabrications, dans le milieu granulaire, ces proportions pouvant également varier avec l'usure des billes et les caractéristiques de filtration du drap qui les recouvre. Par ailleurs, la réalisation de lits avec un milieu granulaire autre que le verre peut remettre en cause la tendance légérement basique du milieu actuel. Enfin, la nécessité de traiter des malades porteurs de souches microbiennes particulièrement résistantes peut apparaître dans l'avenir. Il parait donc souhaitable de contrôler le comportement biochimique du milieu granulaire. Dans le brevet FR.A.1.516.528, il est prévu que la nature ou les caractéristiques du gaz introduit peut être modifiée pour que le gaz lui-même exerce une action thérapeutique (enrichissement de l'air en azote ou en anhydride carbonique). Le document DE-A-2.358.174 prévoit également d'introduire dans le fluide porteur des bactéricides qui combinent l'action mécanique du support avec une action curative. Aucun de ces brevets ne propose toutefois d'agir sur le milieu granulaire.

La présente invention repose sur l'idée qu'il est possible de faire jouer au milieu granulaire inerte de support du patient un rôle thérapeutique bactéricide.

Selon la présente invention, le lit fluidisé à usage thérapeutique comprenant une cuve à l'intérieur de laquelle est introduit un milieu granulaire traversé de bas en haut par un courant vertical de gaz, un drap poreux étant posé sur la surface supérieure dudit milieu grannluire, est caractérisé en ce que des granulés bactéricides dont l'activité biochimique est supérieure à celle du milieu granulaire sont introduits dans ledit milieu grannluire, la densité de la matière constituant les granulés bactéricides étant supérieure à celle de la matière constituant le milieu granulaire.

Il est ainsi possible de contrôler l'action biologique du milieu granulaire en incorporant à celui-ci une proportion plus ou moins grande de granulés bactéricides plus ou moins actifs.

Selon une autre caractéristique de l'invention, les granulés précités sont de préférence des billes. Cette forme particulière contribue à l'obtention d'une bonne fluidisation, les granulés sphériques pouvant se mouvoir au milieu des microbilles de verre.

D'autres caractéristiques et avantages de l'invention apparaîtront au cours de la description qui va suivre de modes de réalisation particuliers, donnés uniquement à titre d'exemples non limitatifs, en regard des dessins qui représentent:
- La Fig.1, un lit fluidisé de type connu;
- La Fig. 2, un exemple de répartition de granulés bactéricides dans le milieu granulaire de microbilles de verre.

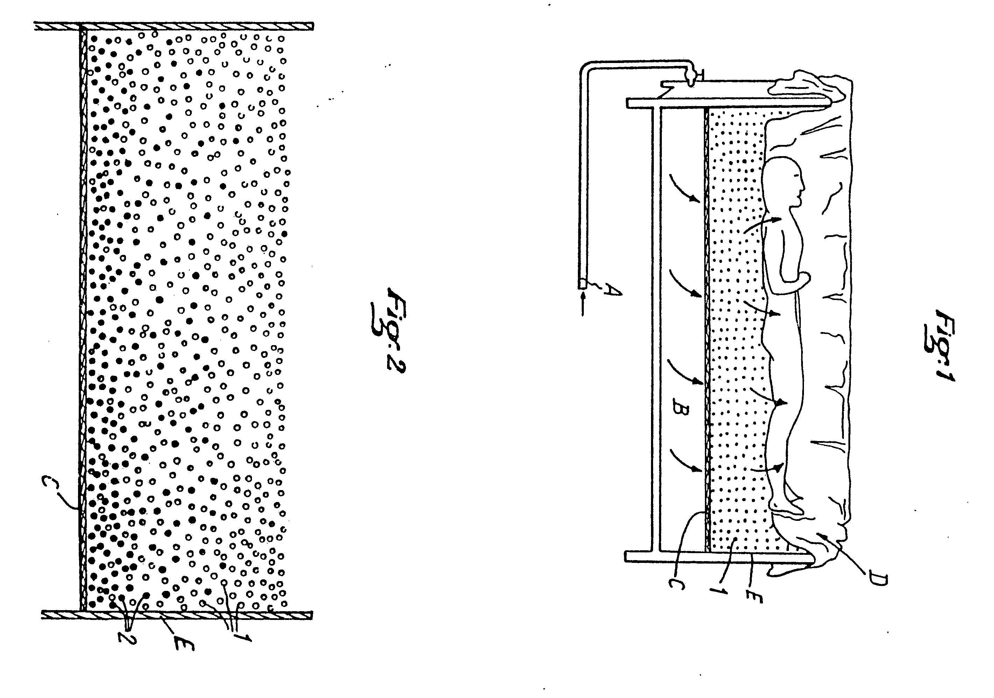

Sur la Fig.1, le lit fluidisé se compose d'une cuve E contenant un milieu granulaire constitué de billes 1 de verre ou de céramique, par exemple. La partie inférieure de la cuve E est constituée par un diffuseur poreux C qui surmonte une chambre de compression 3 recevant un gaz par un ajutage A. La surface supérieure du milieu granulaire est recouverte par un drap filtre D, sur lequel repose le malade. Les fléches indiquent sommairement le sens d'écoulement de l'air ou du gaz. En fait, les microbilles 1 sont soumises à des mouvements de type brownien sous l'action du gaz et de leurs chocs mutuels.

Comme cela a été dit précédemment, l'invention concerne un moyen permettant de contrôler l'activité bactéricide du milieu granulaire en introduisant dans ce milieu des granulés qui sont de préférence de forme sphérique afin de faciliter l'écoulement du gaz d'une part, et un mélange intime des billes de verre et de billes bactéricides d'autre part. De préférence, la dimension des granulés est assez voisine de celle des billes de verre afin que la surface active spécifique par unité de masse soit aussi élevée que possible.

Selon la caractéristique de l'invention, que la densité du matériau constitutif des granulés est légèrement supérieure à celle du matériau constituant les billes inertes, ou évite un contact direct entre le drap supportant le malade et les granulés dont l'activité biochimique pourrait se révéler néfaste.

On a représenté schématiquement sur la Fig. 2 une répartition des granulés 2 à l'intérieur du milieu 1 de microbilles. Sous l'influence de l'agitation provoquée par l'écoulement du gaz et des densités différentes des deux composants du mélange, il s'établit un équilibre de type statistique de sorte qu'il existe un gradient de densités, croissant du haut vers le bas, des granulés dans le milieu de billes de verre. Bien entendu, cet équilibre ne s'établit que lors du fonctionnement en continu de l'insufflation de gaz. Ainsi les granulés se rassemblent à la partie inférieure de la cuve et tout contact avec les plaies du malade est évité. Par ailleurs, l'activité biochimique du matériau constituant les granulés, bien que supérieure à celle des billes de verre, doit être relativement limitée car il faut éviter tout risque d'irritation des plaies par les poussières provenant de l'usure des granulés qui pourraient traverser le drap filtre.

Une recherche systématique des matériaux utilisables pour réaliser les granulés en répondant aux desiderata ci-dessus a conduit aux métaux alcalins tels que le calcium et le magnesium, aux alliages de ces deux métaux entre eux, ainsi qu'avec d'autres éléments plus denses et moins réactifs tels que l'aluminium, le bismuth et le silicium. En effet, le calcium et le magnesium s'oxydent spontanément au contact de l'air et les oxydes ainsi produits forment, en présence d'eau, des bases actives, surtout dans le cas du calcium, mais également dans le cas du magnésium. En réalisant des alliages de ces deux éléments avec des éléments moins actifs tels que ceux cités plus haut, on peut obtenir le niveau d'activité biochimique désiré.

Les billes peuvent être également constituées par un noyau quelconque recouvert d'une couche mince de matières bactéricides.

Les liquidea qui s'écoulent du corps du malade, généralement acides, tombent par gravité vers le bas de la cuve à travers les microbilles de verre qu'ils agglomèrent temporairement. A un moment quelconque de ce parcours, ils entrent en contact avec les granulés et réagissent avec ceux-ci ce qui provoque la formation de sels et la destruction des souches microbiennes.

## Revendications

1°) Lit fluidisé à usage thérapeutique comprenant une cuve (E) à l'intérieur de laquelle est introduit un milieu granulaire (1) traversé de bas en haut par un courant vertical de gaz, un drap poreux (D) étant posé sur la surface supérieure dudit milieu granulaire, caractérisé en ce que des granulés bactéricides (2) dont l'activité biochimique est supérieure à celle du milieu granulaire (1) sont introduits dans ledit milieu grannlaire, la densité de la matière constituant les granulés bactéricides (2) étant supérieure à celle de la matière constituant le milieu granulaire.

2°) Lit fluidisé selon la revendication 1, caractérisé en ce que les granulés bactéricides (2) sont de forme sphérique, leur rayon étant du même ordre de grandeur que celui des billes (1) constituant le milieu granulaire (1).

3°) Lit fluidisé selon l'une des revendications 1 ou 2, caractérisé en ce que les granulés bactéricides (2) sont constitués au moins en partie par au moins un corps choisi dans la classe regroupant le calcium, le magnésium, l'aluminium, le bismuth et le silicium.

## Patentansprüche

1. Tragströmungsbett zur therapeutischen Verwendung mit einer Wanne (E), die mit einem körnigen Medium (1) aufgefüllt ist, das von unten nach oben von einer vertikalen Gasströmung durchdrungen ist, und mit einem porösen Tuch (D), das auf die Oberfläche des körnigen Mediums aufgelegt ist, dadurch gekennzeichnet, daß ein keimtötendes Granulat (2) in das Körnige Medium eingebracht ist, wobei die biochemische Wirksamkeit des keimtötenden Granulats höher ist als die des körnigen Mediums (1) und die Dichte des das keimtötende Granulat (2) bildenden Stoffes größer ist als die des das körnige Medium bildenden Stoffes.

2. Tragströmungsbett nach Anspruch 1, dadurch gekennzeichnet, daß die Bestandteile des keimtötenden Granulats (2) kugelförmig sind und ihr Radius dieselbe Größenordnung aufweist wie die kugelförmigen Körner des körnigen Mediums (1).

3. Tragströmungsbett nach Anspruch 1 oder 2, dadurch gekennzeichnet, daß das keimtötende Granulat (2) wenigstens teilweise aus wenigstens einer der Substanzen Kalzium, Magnesium, Aluminium, Wismut und Silizium gebildet ist.

## Claims

1. Fluidized bed for therapeutic use comprising a tank (E) filled with a particulate material (1) through which passes an upward flow of gas, a porous sheet (D) lying on the upper surface of the said particulate material, characterized in that bactericide particles (2) having a greater biochemical activity than particulate material (1) are introduced in the said particulate material, the density of the bactericide particles (2) being higher than the density of the particulate material (1).

2. Fluidized bed according to claim 1, characterized in that the bactericide particles (2) have a spherical shape the radius of which being of the same order of magnitude as the radius of the spheres constituting the particulate material (1).

3. Fluidized bed according to one of the claims 1 or 2, characterized in that the bactericide particles (2) are made of at least one element selected in the class made of calcium, magnesium, aluminium, bismuth and silicium.

## Fig.1

## Fig.2